# EUROPEAN PATENT SPECIFICATION

(11) **EP 2 482 795 B1**
(45) Date of publication and mention of the grant of the patent: **04.05.2016**
(21) Application number: 09793162.0
(22) Date of filing: 30.09.2009
(51) Int. Cl.: A61K 8/92, A61Q 15/00, A61K 8/36

(54) **ANTIPERSPIRANT COMPOSITION**
ANTIPERSPIRANT-ZUSAMMENSETZUNG
PRÉPARATION ANTI-TRANSPIRANTE

(43) Date of publication of application: 08.08.2012
(73) Proprietor: Colgate-Palmolive Company, New York, NY 10022 (US)
(72) Inventor: FAN, Aixing, Bridgewater NJ 08807 (US); MISNER, H., Steven, Verona NJ 07044 (US); KILPATRICK-LIVERMAN, Latonya, Princeton NJ 08542 (US); LINN, Elizabeth, Lyndhurst NJ 07071 (US); CARLONE, Darrick, Morristown NJ 07960 (US); HOGAN, John, P., Piscataway NJ 08854 (US)
(74) Representative: Jenkins, Peter David
(86) International application number: PCT/US2009/058957
(87) International publication number: WO 2011/040909

(56) References cited:
- EP-A1- 2 281 552
- WO-A1-01/62232
- WO-A2-2010/046012
- FR-A1- 2 270 845
- US-A- 2 087 162
- US-A- 6 001 341
- US-A1- 2002 068 811
- US-A1- 2002 151 453
- US-A1- 2005 281 851
- US-A1- 2008 095 808
- US-A1- 2009 238 786
- US-B1- 6 277 182

## Description

### BACKGROUND OF THE INVENTION

It is known that fatty acids can be selected as a gellant in an antiperspirant/deodorant composition. While fatty acids can be used, they are not used in practice because they result in products that create an undesired level of softness, which results in too much payout of the product. It would be desirable to use fatty acids because of the lower cost, but there needs to be a solution to the softness caused by the fatty acids.

### SUMMARY OF THE INVENTION

The present invention provides an antiperspirant composition according to claim 1.

Preferred features are defined in the dependent claims.

### DETAILED DESCRIPTION

As used throughout, ranges are used as a shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

The present invention includes plant (natural) oils in an antiperspirant composition that overcomes the expectation that the composition will have an oily feel and leave a white residue.

### Fatty Acids

The composition includes a fatty acid. The fatty acid is present in its acid form. While fatty acids are present in plant oils, this is an additional amount of fatty acid that is added. The saturated fatty acid is stearic acid and/or palmitic acid. In one embodiment, the saturated fatty acid is C16. The amount of fatty acid in the composition is greater than 7 weight% and may be up to 30 weight% of the composition . In other embodiments, the amount of fatty acid is at least 7.5, 8, 9, 10, 11, 12, 13, 14, 15, 16, 17, 18, 19, 20, 21, 22, 23, 24, or 25 weight %. In certain embodiments, the amount of saturated fatty acid is 15 to 21 weight%. In other embodiments, the amount of saturated fatty acid is 16 to 20 weight%.

### Plant Oils

The composition includes a plant oil. By plant oil it is meant that the oil is obtained from a plant. The term plant oil does not include fragrances.

In certain embodiments, the plant oil has a melting point below 40°C or below 35°C or below 30°C.

The plant oil is at least one oil chosen from palm kernel oil and coconut oil.

In another embodiment, the plant oil is a combination of palm kernel oil and coconut oil.

The tables below show representative compositions of selected oils.

| Chain Length | Name | Palm Kernel | Coconut | Avocado | Babassu | Canola | Corn |
|---|---|---|---|---|---|---|---|
| C6:0 | caproic | <0.8 | <0.6 | 0 | | 0 | 0 |
| C8:0 | caprylic | 2.4-6.2 | 4.6-10 | 0 | | 0 | 0 |
| C10:0 | capric | 2.6-5 | 5.5-8 | 0 | | 0 | 0 |
| C12:0 | lauric | 45-55 | 45.1-50.3 | 0 | 50 | 0 | 0 |
| C14:0 | myristic | 14-18 | 16.8-21 | 0 | 20 | 0 | 0 |
| C16:0 | palmitic | 6.5-10 | 7.5-10.2 | 5-15 | 11 | 4-5 | 11 |
| C16:1 | palmitoleic | 0 | 0 | 5 | | 0 | 0 |
| C18:0 | stearic | 1-3 | 2-4 | 3 | 3.5 | 1.5-2.5 | 2 |
| C18:1 | oleic | 12-19 | 5-10 | 59-74 | 10 | 53-6 | 28 |
| C18:2 | linoleic | 1-3.5 | 1-2.5 | 10-20 | | 20-23 | 58 |
| C18:3 | alpha linoleic | 0 | 0 | 3 | | 9-12 | 1 |
| C20:0 | arachidic | 0 | 0 | 0 | | 0 | 0 |
| C22:0 | behenic | 0 | 0 | 0 | | 0 | 0 |
| C22:1 | erucic | 0 | 0 | 0 | | <2 | 0 |
| Monounsaturates (C18:1) | | 12 | 6 | 71 | | 62 | 28 |
| Polyunsaturates (C18:2 and C18:3) | | 2 | 2 | 14 | | 32 | 59 |
| Saturates (C 10:0, C12:0, C14:0, C16:0, C18:0) | | 86 | 92 | 12 | | 6 | 13 |
| Iodine value (cg/g) | | 14-21 | 6.3-10.6 | 75-95 | | 110-120 | 120-130 |
| Melting Point (°C) | | 25-30 | 20-28 | <0 | | <0 | <0 |

| Chain Length | Name | Cottonseed | Olive | Palm | Sunflower | | |
|---|---|---|---|---|---|---|---|
| | | | | | Hi-oleic | Mid-oleic | Regular |
| C6:0 | caproic | 0 | 0 | 0 | 0 | No data | 0 |
| C8:0 | caprylic | 0 | 0 | 0 | 0 | No data | 0 |
| C10:0 | capric | 0 | 0 | 0 | 0 | No data | 0 |
| C12:0 | lauric | 0 | 0 | <0.5 | 0 | No data | 0 |
| C14:0 | myristic | 0.6-1 | 0 | 0.5-2 | 0 | No data | 0 |
| C16:0 | palmitic | 21.4-26.4 | 7.5-20 | 39.3-47.5 | 4 | No data | 5-7.6 |
| C16:1 | palmitoleic | 0-1.2 | 0 | 0 | 0 | No data | 0 |
| C18:0 | stearic | 2.1-3.3 | 0.5-5 | 3.5-6 | 6 | No data | 2.7-6.5 |
| C18:1 | oleic | 14.7-21.7 | 55-83 | 36-44 | 85 | 55-65 | 14-39.4 |
| C18:2 | linoleic | 46.7-58.2 | 3.5-21 | 9-12 | 5 | No data | 48.3-74 |
| C18:3 | alpha linoleic | 0-0.4 | <1.5 | <0.3 | 0 | 1 | 0-0.3 |
| C20:0 | arachidic | 0.2-0.5 | <0.8 | 0 | 0 | No data | 0.1-0.5 |
| C22:0 | behenic | 0-0.6 | 0 | 0 | 0 | | 0.3-1.5 |
| C22:1 | erucic | 0 | 0 | 0 | 0 | No data | 0 |
| Monounsaturates (C18:1) | | 19 | 77 | 39 | 85 | 58 | 20 |
| Polyunsaturates (C18:2 and C18:3) | | 55 | 9 | 10 | 5 | 31 | 69 |
| Saturates (C10:0, C12:0, C14:0, C16:0, C18:0) | | 26 | 14 | 51 | 10 | 9 | 11 |
| Iodine value (cg/g) | | 100-115 | 75-94 | 50-55 | 88 | 100-112 | 118-141 |
| Melting Point (°C) | | <0 | <0 | 37.3 | <0 | <0 | <0 |

The amount of plant oil in the composition is at least 12 weights %. In certain embodiments it may be a up to 20 weight% of the composition. In certain embodiments, the amount is greater than 13, 14, 15, 16, 17, 18, or 19 weight % in the composition. In certain embodiments, the amount of plant oil is greater than the amount of volatile silicone in the composition. In one embodiment, there is no volatile silicone in the composition. In other embodiments, the amount of plant oil is more than 10, 15, 20, 25, 30, 35, 40, 45, 50, 55, 60, 65, 70, 75, 80, 85, 90, or 95 weight % of the combined weight of plant oil and volatile silicone (if present).

### Combination of Saturated Fatty Acid and Plant Oil

In certain embodiments, the combination of a saturated fatty acid (in particular a C16 fatty acid) in an amount of 15-21 weight% (or 16-20 weight%) with a plant oil (palm kernel or coconut oil) in an amount of 12-20 weight% provides a structure with a commercially acceptable compression value, which can be at least 3500 g.

### Additional Gellants

In certain embodiments, additional (second) gellants can optionally be included in the composition. Gellants are those materials known in the art that structure the composition. Examples include, but are not limited to, waxes, fatty alcohol, hydrogenated vegetable oil, a hydrocarbon wax, esters of fatty acid and fatty alcohol, triglycerides, or other cosmetically acceptable materials, which are solid or semi solid at room temperature and provide a consistency suitable for application to the skin.

In one embodiment, the hydrogenated oil is hydrogenated soybean oil. In one embodiment, the hydrogenated soybean oil is almost, but not fully hydrogenated. The amount of hydrogenation is measured by the iodine value. The iodine value can be measured by ASTM D5554-95 (2006). In one embodiment, the iodine value of the hydrogenated soybean oil used herein is greater than 0 to 20. In one embodiment, the iodine value is 1 to 5. In another embodiment, the soybean oil is fully hydrogenated with an iodine value of 0. In another embodiment, the iodine value is up to 20. Reference is made to United States Patent Publication No. 2008/0187504A1.

In one embodiment, the gellant includes a partially hydrogenated soybean oil having an iodine value in the range of about 75 to about 80. This partially hydrogenated soybean oil can be obtained from Cargill under the product designation S-500. Reference is made to United States Patent Publication No. 2008/0187503A1. This material has a typical fatty acid distribution shown in the table below. Amounts shown are in % by weight.

| | |
|---|---|
| C16:0 | 10.5-11.2 |
| C18:0 | 6.8-7.5 |
| C18:1 | 61-65 |
| C18:2 | 16-19 |
| C18:3 | 0-0.2 |
| Saturates | 17.5-19.5 |
| Trans | 34-39 |

The hydrocarbon wax can be a hydrocarbon of the formula CₙH₂ₙ₊₂, wherein n is 20-100, and the hydrocarbon is at least 90% linear. In one embodiment, the hydrocarbon is a paraffin. In another embodiment, the hydrocarbon is polyethylene. An example of a polyethylene can be found in United States Patent No. 6,503,491. In another embodiment, the polyethylene has a weight average molecular weight in of about 300 to about 3000 and a melting point of about 50 to about 129°C. In one embodiment, he hydrocarbon is synthetically made from methylene to form a polymethylene.

The fatty alcohol can be any fatty alcohol. In one embodiment, the fatty alcohol is stearyl alcohol.

In another embodiment, the gellant includes hydrogenated castor oil (castor wax). In certain embodiments, the melting point of the castor wax is 70 to 90, or it can be 70, 80, or 90.

In one embodiment, the gellant is a combination of the hydrogenated soybean oil with a fatty alcohol or the hydrocarbon. Reference is made to United States Patent Publication No. 2008/0187504A1.

### Antiperspirant active materials

The composition includes an antiperspirant active. Any of the known antiperspirant active materials can be utilized in the composition. Antiperspirant actives include, but are not limited to, aluminum chlorhydrate, aluminum chloride, aluminum sesquichlorohydrate, aluminum-zirconium hydroxychlorides, complexes or adducts of the above-mentioned active ingredients with glycol, such as propylene glycol (for example, "Rehydrol" II from Reheis Chemical Co.), and combinations thereof. Known aluminum-zirconium salts in combination with neutral amino acids, such as glycine (e.g., aluminum-zirconium tetrachlorohydrex Gly) can also be used. Generally, any of the Category I active antiperspirant ingredients, listed in the Food and Drug Administration's Monograph on Antiperspirant Drug Products for overall-the-counter human use (Oct. 10, 1973) can be used.

In other embodiments, the antiperspirant active is an aluminum salt and/or an aluminum-zirconium salt, such as those described above, that are further stabilized by betaine and a calcium salt. More information about betaine and calcium salt stabilized antiperspirant salts can be found in U.S. Patent Application Publication No. 2006/0204463 to Tang et al.

In other embodiments, the antiperspirant active, such as those described above, is selected to have a low metal to chloride ratio. Examples of these antiperspirant actives can be found in U.S. Patent No. 6,375,937 to Chopra et al. and in U.S. Patent Application Publication No. 2004/0109833 to Tang et al.

In other embodiments, the type of salt of interest, an aluminum zirconium tetrasalt or octasalt free of glycine are used wherein aluminum zirconium salt is stabilized by Betaine and has a metal to chloride ratio of about 0.9:1 to about 1.3:1 (and in other embodiments of about 0.9:1 to about 1.2:1 or about 0.9:1 to about 1.1:1). For the tetrasalt, the Al/Zr atomic ratio can be about 3.2:1 to about 4.1:1.0 and the Betaine:zirconium mole ratio can be about 0.2:1 to about 3.0:1 (or in other embodiments of about 0.4:1 to about 1.5:1). Another salt that can be used is an aluminum chloride salt buffered by Betaine, wherein the salt has a metal to chloride ratio of 0.9:1 to 1.3:1 (and in other embodiments of about 0.9:1 to about 1.2:1 or about 0.9:1 to about 1.1:1). For the octasalt the Al/Zr atomic ratio is about 6.2:1 to about 10.0:1 and the Betaine:Zr mole ratio is about 0.2:1 to about 3.0:1 (or in other embodiments of about 0.4:1 to about 1.5:1). In one embodiment, in the case of a salt that contains zirconium, the Betaine is incorporated during the synthesis of the salt so as to maximize the stabilizing effect this ingredient has (especially on the zirconium species). Alternatively, it can be post added to a glycine-free salt along with additional active phase ingredients to form a Betaine stabilized active.

Examples of commercially available glycine-free low M:Cl ratio tetrasalts and octasalts include, but are not limited to, REZAL™ AZP 955 CPG and REZAL™ AZP 885 respectively (both from Reheis Chemical Company, Berkeley Heights, NJ). A more detailed description of making such commercially available salts can be found for example, in U.S. Patent Nos. 7,074,394 and 6,960,338. Further examples of making these types of salt complexes are described in U.S. Patent Application Publication No. 2004/0198998 and United States Patent No. 7,105,691.

In addition to the anti-irritation properties of Betaine, it has also been found that antiperspirant formulations preserve their fragrance stability upon ageing when the Al/Zr salt is used in association with Betaine.

Additionally, the antiperspirant active can be a calcium salt stabilized antiperspirant active. Examples of calcium salt stabilized antiperspirant actives can be found in U.S. Patent Application Publication No. 2006/0204463.

in addition, any new ingredient, not listed in the Monograph, such as aluminum nitratohydrate and its combination with zirconyl hydroxychlorides and nitrates, or aluminum-stannous chlorohydrates, can be incorporated as an antiperspirant active. Antiperspirant actives can include, but are not limited to, the following: astringent salt of aluminum, astringent salt of zirconium, aluminum bromohydrate, aluminum chlorohydrate, aluminum dichlorohydrate, aluminum sesquichlorohydrate, aluminum chlorohydrex PG, aluminum dichlorohydrex PG, aluminum sesquichlorohydrex PG, aluminum chlorohydrex PEG, aluminum dichlorohydrex PEG, aluminum sesquichlorohydrex PEG, aluminum chloride, aluminum sulfate, aluminum zirconium chlorohydrate, aluminum zirconium trichlorohydrate, aluminum zirconium tetrachlorohydrate, aluminum zirconium pentachlorohydrate, aluminum zirconium octachlorohydrate, aluminum zirconium tetrachlorhydrex propylene glycol, aluminum- zirconium trichlorohydrex Gly, aluminum zirconium tetrachlorohydrex Gly, aluminum zirconium pentachlorohydrex Gly, aluminum zirconium octachlorohydrex Gly, buffered aluminum sulfate, potassium alum, sodium aluminum chlorohydroxy lactate. In one embodiment, the antiperspirant active is aluminum chlorhydrate. In another embodiment, the antiperspirant active is aluminum zirconium tetrachlorhydrex propylene glycol.

### Deodorant active materials

Any known deodorant active can be used. Examples of deodorant active include, but are not limited to, antimicrobial actives, alcohols, 2,4,4'-trichloro-2'-hydroxy diphenyl ether (Triclosan), benzethonium chloride, potyhexamethytene biguanides, triethylcitrate, 2-amino-2-methyl-1-propanol (AMP), cetyl-trimethylammomium bromide, cetyl pyridinium chloride, farnesol (3,7,11-trimethyl-2,6,10-dodecatrien-1-ol), bactericides, and/or bacteriostats.

### Volatile silicone

Compositions according to the present invention can include a volatile silicone. In some embodiments, volatile silicone is excluded from the composition. In one embodiment, the volatile silicone is a volatile cyclic polydimethylsiloxane (cyclomethicone). e.g., cyclopentasiloxane. By volatile material it is meant that the material has a measurably vapor pressure at ambient temperature. Preferably, the volatile cyclic polydimethylsiloxane is cyclomethicone. Various types of cyctomethicones may be used. Illustratively, and not by way of limitation, the volatile silicones are one or more members selected from cyclic polydimethylsiloxanes such as those represented by Formula 1: 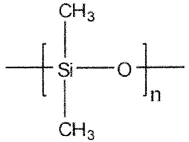 where n is an integer with a value of 3-7, particularly 5-6. Illustrative examples of suitable cyclomethicones are DC-345 and DC-245, manufactured by Dow Corning Corporation, Midland, MI. These types include a tetramer (octylmethylcyclotetrasiloxane) and a pentamer (decamethylcyclopentasiloxane). In one embodiment, the amount of volatile silicone in the composition is greater than 0 up to 40 weight% of the composition. In another embodiment, the amount is less than 40, 35, 30, 25, 20. 15, 10, 5, or 1 weight% of the composition. In one embodiment, there is no volatile silicone in the composition. In another embodiment, there is no silicone in the composition. In another embodiment, the combined amount of the plant oil and volatile silicone is up to 50, 45, 40, 35, 30, 25, or 20 weight%.

### Talc

In certain embodiments, the composition can contain talc. In one embodiment, the amount of talc in the composition is 1 to 10 weight% of the composition.

### Emollients

The composition can contain emollients in any desired amount to achieve a desired emollient effect. Emollients are known in the art and are used to impart a soothing effect on the skin. Non-volatile emollients are preferable in the present invention. Classes of non-volatile emollients include non-silicone and silicone emollients. Non-volatile, non-silicone emollients include C₁₂₋₁₅alkyl benzoate. The non-volatile silicone material can be a polyethersiloxane, polyalkyarylsiloxane or polyethersiloxane copolymer. An illustrative non-volatile silicone material in the present invention is phenyl trimethicone. Noun-limiting examples of emollients can be found in United States Patent No. 6,007,799. Examples include, but are not limited to, PPG-14 butyl ether, PPG-3 myriscyl ether, stearyl alcohol, stearic acid, glyceryl monorieinoleate, isobutyl palmitate, glyceryl monostearate, isocetyl stearate, sulphated tallow, oleyl alcohol, propylene glycol, isopropyl laurate, mink oil, sorbitan stearate, cetyl alcohol, hydrogenated castor oil, stearyl stearate, hydrogenated soy glycerines, isopropyl isostearate, hexyl laurate, dimethyl brassylate, decyl oleate, diisopropyl adipate, n-dibutyl sebacate, diisopropyl sebacate. 2-ethyl hexyl palmitate, isononyl isononanoate, isodecyl isononanoate, isotridecyl isononanoate, 2-methyl hexyl palmitate, 2-ethyl hexyl stearate, Di-(2-ethyl hexyl) adipate), Di-(2-ethyl hexyl) succinate, isopropyl myristate, isopropyl palmitate, isopropyl stearate, octacosanol, butyl stearate, glyceryl monostearate, polyethylene glycols, oleic acid, triethylene glycol, lanolin, castor oil, acetylated lanolin alcohol, acetylated lanolin, petrolatum, isopropyl ester of lanolin, fatty acids, mineral oils, butyl myristate, isostearic acid, palmitic acid. PEG-23 oleyl ether, olelyl oleate, isopropyl linoleate, cetyl lactate, lauryl lactate, myristyl lactate, quaternised hydroxy alkyl, aminogluconate, vegetable oils, isodecyl oleate, isostearyl neopentanoate, myristyl myristate, oleyl ethoxy myristate, diglycol stearate, ethylene glycol monostearate, myristyl stearate, isopropyl lanolate, paraffin wares, glycyrrhizic acid, hydrocyethyl stearate amide.

The composition can additionally include ionizable inorganic salts. These ionizable salts are of the form MₐX_{b} where a=1, or 2 and b=1 or 2; M is a member chosen from Na⁺¹, Li⁺¹, K⁺¹, Mg⁺², Ca⁺², Sr⁺², and Zn⁺² and X is a member chosen chloride, bromide, iodide, citrate, gluconate, lactate, glycinate, glutamate, ascorbate, aspartate, nitrate, phosphate, hydrogenphosphate, dihydrogenphosphate, formate, maloneate, maleate, succinate, carbonate, bicarbonate, sulfate, and hydrogensulfate. In certain embodiments, the selected salts are chosen from NaCl and ZnCl₂. As will be appreciated by those skilled in the art, while it may be possible under certain circumstances to add a salt directly to a portion of the mixture during manufacturing, it is desired to add the salt as a mixture or solution of the salt in a carrier or solvent, particularly water. Of course various concentrations of the salt premix can be made.

The composition may also contain particulates which include but are not limited to talc, mica, fragrance encapsulates, or hydrophobically modified starches, such as aluminum starch octenyl succinate (MACKADERM™ ASTRO-DRY™ from McIntyre Group Ltd.). If the composition is in a liquid form and dispensed through a roll-on applicator, the average particle size of the suspended material is sized so that it can pass through the application to prevent the ball applicator from malfunctioning. Usually, the average particle size does not exceed 150 microns.

In certain embodiments, the composition may also contain as an optional ingredient at least one malodor counteracting alpha, beta-unsaturated ester or mixtures of such materials. In certain embodiments, the level of malodor counteracting composition to deliver a perceivable odor control benefit when deliverer from an antiperspirant and/or deodorant composition is about 0.05 to about 0.45 weight % based on the entire compositions. The alpha, beta-unsaturated ester malodor counteracting materials are incorporated within the oil phase of an antiperspirant composition. Example of these malodor counteracting components can be found in U.S. Patent No. 6,610, 648 and U.S. Patent No. 6,495,097. For example, in this invention the odor neutralizing alpha, beta unsaturated ester mixture demonstrates unexpected stability in antiperspirant compositions containing low metal:chloride (M:Cl) ratio salts free of glycine. Examples of the alpha, beta unsaturated ester can be found in WO2005/025523, which was filed in the United States as U.S. Application No. 10/571,488.

Examples of the alpha, beta unsaturated ester include, but are not limited to,:
(1) 3-phenyl-2-propenoic acid alkyl esters wherein R¹ is a substituent on the benzene ring and is chosen from an alkyl, an alkoxy, an aryl, or a substituted aryl. In certain embodiments, R¹ is chosen from H, a C₁ to C₈ alkyl, a C₁ to C₈ alkoxy, or an aryl; and R² is a subsistent group replacing the carboxylic acid hydrogen to form the ester where R² has greater than 6 carbon atoms, an aryl, or a substituted aryl group, in certain embodiments R² is a C₆ to C₁₂ alkyl or is a benzyl group; and
(2) an ester of fumaric or maleic acid having linear ester carbon chains from 3-9 carbons, for example dihexyl fumarate;
(3) e-phenyl propenoic acid ester chosen from octyl methoxy cinnamate, phenylethyl cinnamate, benzyl cinnamate;
(4) an aliphatic unsaturated ester, such as dihexyl fumarate.

The composition may optionally further comprise absorbent materials such as corn starch, talc, clay, sodium polyacrylate and/or cotton fiber; and/or other materials such as fragrances, bacteriostats and/or bacteriosides, colorants, etc. Known bacteriostats include baceteriostatic quaternary ammonium compounds such as 2-amino-2-methyl-1-propanol (AMP), cetyl-trimethylammomium bromide, cetyl pyridinium chloride, 2,4,4N-trichloro-2N-hydroxydiphenylether (Triclosan), etc. and various zinc salts.

Antioxidants may be added to the composition, preferably to act as ingredient protectants and for maintenance of long-term stability of the composition. Suitable antioxidants include Tinogard, manufactured by Ciba Specialty Chemicals, Basel, Switzerland.

The compositions as provided herein are described and claimed with reference to their ingredients, as is usual in the art. As would be evident to one skilled in the art, ingredients may in some instances react with one another, so that the true composition of the final formulation may not correspond exactly to the ingredients listed. Thus, it should be understood that the invention extends to the product of the combination of the listed ingredients.

The compositions of the present invention may be manufactured using methods known in the art. Typically, the ingredients are combined and heated to melt the components (other than inert filler), and the melted components (together with particulate inert filler) are mixed. Desirably, volatile materials, such as the fragrance materials, are incorporated in the composition in the latter stages of the mixing cycle, in order to avoid volatilization thereof. After mixing, the molten composition can be poured directly into the dispensers, after which the compositions harden into a solid, and the container is capped to preserve the product until use.

The composition is a solid stick when at ambient room temperature of about 25°C. The stick form is an example of a solid form. The stick form can be distinguished from a soft solid in that, in a stick, the formulated product can retain its shape for extended time periods outside the package, the product not loosing its shape significantly (allowing for some shrinkage due to solvent evaporation). Adjustment of amounts of gelling or thickening agents can be used in order to form a stick.

Reference is made to U.S. Pat. No. 5,102,656, U.S. Pat. No. 5,069,897, and U.S. Pat. No. 4,937,069.

In one embodiment, the composition is an anhydrous stick. By anhydrous it is meant that no separate water is added but there could be moisture associated with materials that are added to the composition. In certain embodiments, the amount of water is zero or less than 3, 2, 1, 0.5, or 0.1 weight % of the composition.

In one embodiment, the compression force of the composition is at least 3500g. In other embodiments, the compression force is at least 4000g, at least 4500g, at least about 5000g, at least 6000g, at least 7000g, at least about 8000g, at least 9000g. In another embodiment, the compression force is 3500g to 10,000g.

In one embodiment, the composition can provide a payout of about 0.7 to about 0.9g according to the payout test on the Payout, Glide, and Flakeoff Test Machine, which is the machine and method described in WO2009/045557. In another embodiment, the composition can provide a glide of about 0.8 to about 1.4g according to the glide test on the Payout, Glide, and Flakeoff Test Machine. In anther embodiment, the composition can provide a flakeoff of less that about 25%. In other embodiments, the flake off is less than about 20, about 15, about 10, or about 5%. In other embodiments, the amount of flakeoff is about 1 to about 6%.

Compression strength of a stick product is measured using a Texture Analyzer Model # TA-ZT21 from Texture Technologies. The compression probe is a 19 mm square end probe. A 42.5 g (1.5 oz) antiperspirant stick is selected. The antiperspirant stick is removed from the barrel and placed in a hardness sample holder. The stick is positioned such that 2.54cm (1 inch) of the sample, measured at the edge of the domed portion, is exposed for the test. The cover on the hardness holder is closed and the holder positioned so that the blade comes in contact with the midpoint of the exposed sample. The instrument is set to the following parameters:
Measured Force - compression (speed set at 1.0 mm/s)
Option - RETURN TO START
Distance - 5.0 mm
Unit selection - grams.
The measurements to be recorded are peak force and distance required to break the stick. The higher the force reading, the stronger the stick. The longer the distance to break, the more elastic the stick.

### SPECIFIC EMBODIMENTS OF THE INVENTION

The invention is further described in the following examples. The examples are merely illustrative and do not in any way limit the scope of the invention as described and claimed.

The stick compositions bellow are made by standard methods of melting the materials and mixing them together. The comparative examples are typical antiperspirant stick compositions. The Examples use the high levels of fatty acid in combination with plant oil.

| Material | Comparative 1 | 1 | 2 | Comparative 2 | 3 |
|---|---|---|---|---|---|
| C12-15 Alkyl Benzoate | 12 | 0 | 0 | 12 | 0 |
| Stearyl Alcohol | 20 | 0 | 0 | 20 | 0 |
| Castor Wax (80°C melt point) | 4 | 4 | 4 | 4 | 4 |
| PEG-8 distearate | 4 | 4 | 4 | 4 | 4 |
| Aluminium Zirconium Tetrachlorohydrex Gly (Z576 from Summit) | 22 | 22 | 22 | 22 | 22 |
| Cyclomethicone | 37 | 29 | 29 | 36.3 | 28.5 |
| Fragrance | 1 | 1 | 1 | 1.5 | 1.5 |
| Palmitic Acid | 0 | 20 | 20 | 0 | 20 |
| Coconut Oil | 0 | 20 | 0 | 0 | 20 |
| Palm Kernel Oil | 0 | 0 | 20 | 0 | 0 |
| Compression (g) | 2968 | 2638 | 2740 | 3682 | 3162 |

The results show that sticks of comparable hardness can be obtained using fatty acid as a gellant. Also, flake off measurements were run on Example 1 and Comparative 1. Example 1 had a flake off of 7±2 and Comparative 1 had a flake off of 15±2. The results show that high fatty acid level gellant can be used and still deliver low levels of flake off.

The stick compositions below are made by standard methods of melting the materials and mixing them together. The compositions below contain talc.

| Material | Comparative 3 | 4 | 5 |
|---|---|---|---|
| Cyclomethicone | 35 | 30 | 30 |
| Aluminum Zirconium Tetrachlorohydrex Gly (AZP908 from Reheis) | 20 | 20 | 20 |
| Stearyl Alcohol | 16 | 0 | 0 |
| C12-15 Alkyl Benzoate | 10 | 0 | 0 |
| PPG-14 Butyl Ether | 6 | 0 | 0 |
| Castor Wax (80°C melt point) | 5 | 5 | 5 |
| Hydrogenated Soybean Oil iodine value up to 20 | 2 | 2 | 2 |
| PEG-8 Distearate | 3 | 3 | 3 |
| Talc | | 2 | 2 |
| Coconut Oil | 0 | 21 | 0 |
| Palm Kernel Oil | 0 | 0 | 21 |
| Palmitic Acid | 0 | 16 | 16 |
| Fragrance | 1 | 11 | 1 |
| Compression (g) | 1125 | 2352 | 2703 |

Below are stick examples (not according to the invention) using stearic acid and are made by standard methods of melting the material and mixing them together.

| Material | 6 | 7 | 8 |
|---|---|---|---|
| PPG-14 Butyl Ether | 10 | 10 | 10 |
| Stearic Acid | 10 | 8 | 19 |
| Hydrogenated Soybean Oil iodine value up to 20 | 6 | 6 | 0 |
| Castor wax (80°C melt point) | 4 | 4 | 4 |
| PEG-8 Distearate | 4 | 4 | 4 |
| Stearyl Alcohol | 15 | 16 | 9 |
| Behenyl Alcohol | 0.153 | 0.153 | 0.153 |
| Cyclomethicone | 29 | 30 | 32 |
| Aluminum Zirconium Tetrachlorohydrex Gly (AZP908 from Reheis) | 21.847 | 21.847 | 21.847 |
| Compression (g) | 7441 | 4354 | 3729 |

## Claims

1. An antiperspirant composition comprising:
a) an antiperspirant active;
b) a fatty acid in an amount of greater than 7 weight%, wherein the fatty acid is at least one fatty acid chosen from palmitic acid and stearic acid; and
c) a plant oil in an amount of at least 12 weight%, wherein the plant oil is at least one oil chosen from palm kernel and coconut oil,
wherein the composition is a stick product.

2. The composition of claim 1, wherein the amount of the fatty acid is greater than 7 weight% up to 30 weight%.

3. The composition of claim 1, wherein the fatty acid comprises a combination of palmitic acid and stearic acid.

4. The composition of claim 1 further comprising at least one gellant selected from hydrogenated soybean oil, partially hydrogenated soybean oil, a hydrocarbon of the formula CₙH₂ₙ+2, wherein n is 20-100, and the hydrocarbon is at least 90% linear, hydrogenated castor oil, and a fatty alcohol; optionally wherein the gellant comprises hydrogenated soybean oil and a fatty alcohol.

5. The composition of claim 1, wherein there is no silicone in the composition.

6. The composition of claim 1, wherein the composition is a stick product that comprises
a) the antiperspirant active,
b) the fatty acid comprises palmitic acid, and
c) the plant oil comprises palm kernel oil.

7. The composition of claim 1 or claim 6, wherein the amount of plant oil is greater than the amount of volatile silicone.

8. The composition of claim 1 or claim 6, wherein the composition does not include a volatile silicone.

9. A method comprising applying the composition of claim 1 to an axillary area.

## Patentansprüche

1. Antitranspirant-Zusammensetzung, die Folgendes umfasst:
a) einen Antitranspirant-Wirkstoff;
b) eine Fettsäure in einer Menge von mehr als 7 Gew.-%, wobei die Fettsäure mindestens eine Fettsäure ist, die aus Palmitinsäure und Stearinsäure ausgewählt ist; und
c) ein Pflanzenöl in einer Menge von mindestens 12 Gew.-%, wobei das Pflanzenöl mindestens ein Öl ist, das aus Palmkernöl und Kokosöl ausgewählt ist,
wobei die Zusammensetzung ein Stiftprodukt ist.

2. Zusammensetzung nach Anspruch 1, wobei die Menge der Fettsäure mehr als 7 Gew.-% bis zu 30 Gew.-% beträgt.

3. Zusammensetzung nach Anspruch 1, wobei die Fettsäure eine Kombination von Palmitinsäure und Stearinsäure umfasst.

4. Zusammensetzung nach Anspruch 1, die weiterhin mindestens ein Geliermittel umfasst, das aus hydriertem Sojaöl, partiell hydriertem Sojaöl, einem Kohlenwasserstoff der Formel CₙH₂ₙ₊₂, wobei n 20-100 ist und der Kohlenwasserstoff zu mindestens 90 % linear ist, hydriertem Rizinusöl und einem Fettalkohol ausgewählt ist; gegebenenfalls wobei das Geliermittel hydriertes Sojaöl und einen Fettalkohol umfasst.

5. Zusammensetzung nach Anspruch 1, wobei kein Silikon in der Zusammensetzung vorhanden ist.

6. Zusammensetzung nach Anspruch 1, wobei die Zusammensetzung ein Stiftprodukt ist, das Folgendes umfasst:
a) den Antitranspirant-Wirkstoff,
b) die Fettsäure umfasst Palmitinsäure und
c) das Pflanzenöl umfasst Palmkernöl.

7. Zusammensetzung nach Anspruch 1 oder 6, wobei die Menge an Pflanzenöl höher als die Menge an flüchtigem Silikon ist.

8. Zusammensetzung nach Anspruch 1 oder 6, wobei die Zusammensetzung kein flüchtiges Silikon enthält.

9. Verfahren, das das Anwenden der Zusammensetzung nach Anspruch 1 auf einen Achselbereich umfasst.

## Revendications

1. Une composition d'un actif anti-transpirant comprenant:
a) un actif anti-transpirant ;
b) un acide gras dans une quantité supérieure à 7% en poids, dans laquelle l'acide gras est au moins un acide gras choisi parmi l'acide palmitique et l'acide stéarique ; et
c) une huile végétale dans une quantité d'au moins 12% en poids, dans laquelle l'huile végétale est au moins une huile choisie parmi l'huile de noix de coco et l'huile de palmiste,
dans laquelle la composition est un produit en forme de bâton.

2. La composition selon la revendication 1, dans laquelle la quantité de l'acide gras est supérieure à 7% en poids et jusqu'à 30% en poids.

3. La composition selon la revendication 1, dans laquelle l'acide gras comprend une combinaison de l'acide palmitique et de l'acide stéarique.

4. La composition selon la revendication 1 comprenant en outre au moins un gélifiant sélectionné parmi l'huile de soja hydrogénée, l'huile de soja partiellement hydrogénée, un hydrocarbure de la formule CₙH₂ₙ+₂, dans laquelle n est compris entre 20 et 100, et l'hydrocarbure est linéaire à au moins 90%, l'huile de ricin hydrogénée et un alcool gras ; en option dans laquelle le gélifiant comprend l'huile de soja hydrogénée et un alcool gras.

5. La composition selon la revendication 1, dans laquelle il n'y a pas de silicone dans la composition.

6. La composition selon la revendication 1, dans laquelle la composition est un produit en forme de bâton qui comprend :
a) l'actif anti-transpirant,
b) l'acide gras comprend l'acide palmitique, et
c) l'huile végétale comprend l'huile de palmiste.

7. La composition selon la revendication 1 ou la revendication 6, dans laquelle la quantité d'huile végétale est supérieure à la quantité de silicone volatile.

8. La composition selon la revendication 1 ou la revendication 6, dans laquelle la composition n'inclut pas de silicone volatile.

9. Un procédé comprenant l'application de la composition selon la revendication 1 à une zone axillaire.
